(19) Europäisches Patentamt
European Patent Office
Office européen des brevets

(11) **EP 3 265 226 B1**

(12) **FASCICULE DE BREVET EUROPEEN**

(45) Date de publication et mention de la délivrance du brevet:
**06.05.2020 Bulletin 2020/19**

(21) Numéro de dépôt: **16705068.1**

(22) Date de dépôt: **09.02.2016**

(51) Int Cl.:
*B01J 37/10* (2006.01)  *B01J 37/14* (2006.01)
*B01J 23/66* (2006.01)  *B01J 23/52* (2006.01)
*B01J 35/08* (2006.01)  *C10G 45/40* (2006.01)
*B01J 23/44* (2006.01)  *B01J 35/00* (2006.01)
*B01J 37/02* (2006.01)  *B01J 37/06* (2006.01)
*B01J 37/03* (2006.01)  *B01J 37/18* (2006.01)
*B01J 35/02* (2006.01)  *B01J 35/10* (2006.01)
*C07C 5/09* (2006.01)  *C07C 7/167* (2006.01)

(86) Numéro de dépôt international:
**PCT/EP2016/052695**

(87) Numéro de publication internationale:
**WO 2016/139033 (09.09.2016 Gazette 2016/36)**

(54) **CATALYSEUR COMPRENANT DE L'OR DISPERSE ET DU PALLADIUM ET SON APPLICATION EN HYDROGENATION SELECTIVE**

KATALYSATOR MIT DISPERGIERTEM GOLD UND PALLADIUM SOWIE VERWENDUNG DAVON IN SELEKTIVER HYDRIERUNG

CATALYST COMPRISING DISPERSED GOLD AND PALLADIUM AND APPLICATION THEREOF IN SELECTIVE HYDROGENATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.03.2015 FR 1551872**

(43) Date de publication de la demande:
**10.01.2018 Bulletin 2018/02**

(73) Titulaire: **IFP Energies nouvelles**
**92500 Rueil-Malmaison (FR)**

(72) Inventeurs:
• **CABIAC, Amandine**
**69700 Givors (FR)**
• **HUGON, Antoine**
**69700 Givors (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**FR-A1- 2 347 095     FR-A1- 2 932 177**
**US-B1- 6 509 292**

• **Pavani Malla: "DESIGNING SUPPORTED PALLADIUM-ON-GOLD BIMETALLIC NANO-CATALYSTS FOR CONTROLLED HYDROGENATION OF ACETYLENE IN LARGE EXCESS OF ETHYLENE", Thesis for the obtention of the degree of master of science of Texas A&M university , 31 août 2014 (2014-08-31), XP055241153, Extrait de l'Internet: URL:http://dmc.tamuc.edu/cdm/ref/collection/p15778coll7/id/295 [extrait le 2016-01-13]**

**Description**

**Domaine technique**

La présente invention concerne notamment le domaine des catalyseurs hétérogènes, et plus particulièrement des catalyseurs comprenant du palladium et de l'or supportés. L'objet de l'invention est de proposer un catalyseur, notamment en hydrogénation sélective, ainsi qu'un mode de préparation de ce catalyseur.

**Etat de la technique**

Le procédé d'hydrogénation sélective permet de transformer les composés polyinsaturés des coupes pétrolières par conversion des composés les plus insaturés vers les alcènes correspondants en évitant la saturation totale et donc la formation des alcanes correspondants. Les catalyseurs d'hydrogénation sélective sont souvent à base de palladium, sous forme de petites particules métalliques déposées sur un support qui peut être un oxyde réfractaire. La teneur en palladium et la taille des particules de palladium font partie des critères qui ont une importance sur l'activité et la sélectivité des catalyseurs.

Les catalyseurs à base de palladium sont largement utilisés dans les réactions d'hydrogénation sélective de coupes légères C2-C4. Typiquement, le palladium est réparti dans une croute en périphérie du catalyseur (catalyseur egg-shell selon la terminologie anglo-saxonne). Ces dernières années ont vu l'introduction de catalyseurs bimétalliques qui permettant un gain notable en activité, mais surtout en sélectivité dans l'hydrogénation des acétyléniques et des dioléfines. Typiquement, l'élément ajouté au palladium est l'argent ou l'or.

Le document US2006/025302 divulgue un catalyseur pour l'hydrogénation sélective de l'acétylène et de dioléfines, comprenant du palladium, avec une teneur comprise entre 0,01 et 0,1% poids par rapport au poids total du catalyseur, et un métal du groupe IB, avec une teneur comprise entre 0,005 et 0,06% en poids par rapport au poids total du catalyseur. Le métal est de préférence de l'argent. Le palladium est réparti de telle façon que 90% du palladium introduit dans le catalyseur est compris dans une croute inférieure à 250 µm.

Le document US6509292 divulgue un catalyseur pour l'hydrogénation sélective de l'acétylène comprenant du palladium avec une teneur de 0,001% à 0,028% en poids par rapport au poids total du catalyseur et de l'or avec une teneur de 0,18 % à 1% en poids par rapport au poids total du catalyseur. Le rapport or / palladium est compris entre 6:1 et 50:1.

Le palladium est réparti de telle façon que 90% du métal soit compris dans une croute inférieure à 250 µm.

Le document US6350717 décrit un catalyseur comprenant au moins un métal appartenant aux métaux de la colonne 10 selon la classification IUPAC (par exemple la palladium) et au moins un métal appartenant aux métaux de la colonne 11 (par exemple l'or) sur un support en oxyde d'alumine (aluminium), le métal appartenant à la colonne 10 étant essentiellement concentrée à la surface du support, et le métal appartenant à la colonne 11 étant distribuée essentiellement uniformément dans tout le volume du catalyseur, le ratio en poids entre le métal appartenant au groupe 11 et le métal appartenant au groupe 10 ne dépassant pas 1,95.

Le document FR2347095 divulgue un catalyseur d'hydrogénation sélective comprenant de l'or, du palladium, et un support poreux, le palladium étant réparti dans une croûte en périphérie du support poreux. Cependant, ce document reste silencieux sur la manière dont l'or est réparti sur le support poreux, et ne décrit pas la taille des particules d'or.

La thèse de Pavani Malla intitulée : « Designing supported palladium-on-gold bimetallic nano-catalysts for controlled hydrogenation of acetylene in large excess of ethylene », Texas A&M University, Août 2014, divulgue un procédé de préparation d'un catalyseur d'hydrogénation sélective à base de palladium et d'or, lequel procédé comprend entre autre une étape d'imprégnation d'un précurseur de palladium sur un support poreux, une étape d'imprégnation d'un précurseur d'or sur un support poreux. Cependant, ce document ne divulgue pas d'étape spécifique dans laquelle on met en contact le précurseur de catalyseur, après l'étape d'imprégnation du précurseur d'or sur le support poreux, avec une solution contenant de l'urée.

Plus particulièrement, ces types de catalyseurs sont utilisés pour l'hydrogénation sélective de coupes légères C2-C4 en tête de réacteur (en configuration front end selon la terminologie anglo-saxonne). Cette configuration se caractérise par la présence dans la charge d'un large excès d'hydrogène et d'une teneur variable en monoxyde de carbone, entre environ 100 ppm mol et 2000 ppm mol. Le monoxyde de carbone (CO) s'adsorbe partiellement sur le palladium. Lorsque la teneur en CO augmente ou diminue dans une charge C2-C4, l'activité des catalyseurs d'hydrogénation sélective en particulier de catalyseurs contenant du palladium est modifiée.

La demanderesse a constaté qu'il est possible de fournir un catalyseur supporté à base de palladium et d'or, l'or et le palladium étant majoritairement sous forme de particules indépendantes (i.e. la quantité d'alliage palladium-or est minoritaire), l'or se présentant sous forme de petites particules réparties de manière homogène dans le support, le palladium étant réparti en croute en périphérie du support, permettant une amélioration de la stabilité dudit catalyseur vis-à-vis des changements de concentration de CO dans la charge. Par ailleurs, la stabilité du catalyseur selon l'invention est favorisée par la présence d'un nombre important de sites métalliques d'or, obtenues pour des catalyseurs contenant une teneur massique importante d'or ; l'or étant dispersé à l'échelle nanométrique au sein du support.

**Objets de l'invention**

Un premier objet selon l'invention concerne un catalyseur comprenant de l'or, du palladium, un support poreux, se présentant sous la forme d'au moins un grain, dans lequel :

- la teneur en or dans le catalyseur est comprise entre 0,5 et 3% poids par rapport au poids total du catalyseur ;
- la taille moyenne des particules d'or estimée par microscopie électronique à transmission (MET) est comprise entre 0,5 nm et 5 nm ;
- l'or est réparti de manière homogène dans ledit support poreux ;
- au moins 80% poids du palladium est réparti dans une croûte à la périphérie dudit support poreux;
- le ratio molaire or/palladium est supérieur à 2.

De préférence, la taille moyenne des particules d'or estimée par microscopie électronique à transmission est comprise entre 0,5 nm et à 3 nm.
Avantageusement, la dispersion métallique D de l'or est comprise entre 30 et 100 %.
De préférence, la teneur en palladium est comprise entre 0,01 et 0,6% poids par rapport au poids total du catalyseur.
L'épaisseur de ladite croute à la périphérie du support poreux est inférieure à 300 μm.
Un autre objet concerne un procédé de préparation d'un catalyseur selon l'invention comprenant de l'or, du palladium, un support poreux, se présentant sous la forme d'au moins un grain, lequel procédé comprenant les étapes suivantes :

- une étape dans laquelle on introduit le palladium sur le support, dite étape 1, comprenant les étapes suivantes :

    1a) on prépare une solution aqueuse d'oxyde de palladium ou d'hydroxyde de palladium ;
    1b) on imprègne ladite solution sur au moins un grain de support poreux ;
    1c) on soumet le support poreux imprégné obtenu à l'étape 1b) à une maturation afin d'obtenir un précurseur de catalyseur ;
    1d) on sèche le précurseur de catalyseur obtenu à l'étape 1c) à une température comprise entre 50°C et 250°C ;
    1e) on calcine le précurseur de catalyseur obtenu à l'étape 1d) à une température comprise entre 250°C et 900°C ;

- une étape dans laquelle on introduit l'or sur le support, dite étape 2, comprenant les étapes suivantes :

    2a) on prépare une solution aqueuse contenant un précurseur d'or ;
    2b) on imprègne ledit support poreux avec la ladite solution obtenue à l'étape 2a);
    2c) on soumet le support poreux imprégné obtenu à l'étape 2b) à une maturation afin d'obtenir un précurseur de catalyseur ;
    2d) on met en contact le précurseur de catalyseur obtenu à l'étape 2c) avec une solution contenant de l'urée ;
    2e) on sèche le précurseur de catalyseur obtenu à l'étape 2d) à une température comprise entre 50°C et 300°C.

De préférence, on prépare à l'étape 1a) une suspension colloïdale d'oxyde de palladium ou d'hydroxyde de palladium en phase aqueuse.
Avantageusement, on réalise à l'étape 1b) et/ou 2b) une imprégnation à sec.
Les étapes 1c) et 2c) de maturation sont réalisées pendant une durée comprise entre 0,5 et 40 heures.
De préférence, on réalise entre l'étape 2c) et 2d) une étape de séchage dudit précurseur de catalyseur obtenu à l'étape 2c) à une température comprise entre 50°C et 300°C.
Le volume de la solution aqueuse contenant de l'urée préparée à l'étape 2d) est compris entre 0,9 et 20 fois le volume poreux total du support poreux mis en forme.
Le rapport molaire urée/or est compris entre 1 et 1000.
On réalise en outre une étape 2f) dans laquelle on soumet le catalyseur séché obtenu à l'issue de l'étape 2e) à un traitement réducteur par mise en contact avec un gaz réducteur.
Avantageusement, l'étape 2f) de réduction est réalisée à une température comprise entre 40°C et 500°C.
Un autre objet concerne un procédé d'hydrogénation sélective d'une coupe C2-C4 avec un catalyseur selon l'invention ou préparé selon le procédé de l'invention dans lequel la température est comprise entre 0 et 500°C, la pression est comprise entre 0,1 et 20 MPa, la vitesse volumique horaire est comprise entre 0,1 et 50 h$^{-1}$ pour une charge liquide, entre 500 et 30 000 h$^{-1}$ pour une charge gazeuse.

**Description détaillée de l'invention**

Dans la suite, les groupes d'éléments chimiques sont donnés selon la classification CAS (CRC Handbook of Chemistry and Physics, éditeur CRC press, rédacteur en chef D.R. Lide , 81ème édition, 2000-2001). Par exemple, le groupe IB selon la classification CAS correspond aux métaux de la colonne 11 selon la nouvelle classification IUPAC.

Catalyseur

L'invention concerne un catalyseur comprenant un support poreux, se présentant sous la forme d'au moins un grain, de l'or, du palladium, dans lequel :

- la teneur en or dans le catalyseur est comprise entre 0,5 et 3% poids;
- la taille moyenne des particules d'or estimée par microscopie électronique à transmission (MET) est comprise entre 0,5 nm et 5 nm, de préférence inférieure à 4 nm, et encore plus préférentiellement inférieure à 3,5 nm ; et de manière encore plus préférée inférieure à 3 nm ;
- l'or est réparti de manière homogène dans le support poreux ;
- au moins 80% poids du palladium est réparti dans une croûte à la périphérie du support poreux;
- le ratio molaire or/palladium est supérieur à 2, de préférence supérieur à 4, et encore plus préférentiellement supérieur à 5.

Selon l'invention, on entend par grain, le support poreux mis en forme sous forme de billes ou d'extrudés.

L'épaisseur de ladite croûte à la périphérie du support poreux est inférieure à 300 $\mu$m, de préférence inférieure à 250 $\mu$m.

Avantageusement, la teneur en palladium est comprise entre 0,01 et 0,6% poids par rapport au poids total du catalyseur, de préférence compris entre 0,01 et 0,3% poids, et encore plus préférentiellement compris entre 0,03 et 0,3% poids ; de manière encore plus préférée entre 0,035 et 0,2% poids, et encore plus préférentiellement entre 0,04 et 0,2% poids.

La teneur en chlore résiduel est inférieure à 200 ppm poids par rapport au poids total du catalyseur, de préférence inférieure à 100 ppm poids, et encore plus préférentiellement inférieure à 50 ppm poids.

L'or est réparti de manière homogène dans le support poreux, avec un coefficient R (décrit par la suite) compris entre 0,8 et 1,2.

La dispersion D (décrit par la suite) de l'or, i.e. la quantité d'or surfacique par rapport à l'or totale introduit, est comprise entre 30% et 100%, de manière préférée entre 35% et 100%, et de manière très préférée entre 38% et 100%.

Le catalyseur peut comprendre en outre un élément du groupe IB (autre que l'or), de préférence choisi parmi l'argent et le cuivre. De manière préférée, l'élément du groupe IB est l'argent. La teneur en élément du groupe IB est comprise entre 0,01 et 0,3% poids par rapport au poids total du catalyseur, de préférence comprise entre 0,015 et 0,2% poids.

Le catalyseur peut comprendre au moins un métal sélectionné dans le groupe constitué par les alcalins et les alcalino-terreux. La teneur en métal sélectionné dans le groupe constitué par les alcalins et les alcalino-terreux est avantageusement comprise entre 0,02% et 0,5% poids par rapport au poids total du catalyseur.

Le métal alcalin est généralement sélectionné dans le groupe constitué par le lithium, le sodium, le potassium, le rubidium et le césium, de préférence par le lithium, le sodium et le potassium, de manière très préférée par le sodium et le potassium.

Le métal alcalino-terreux est généralement sélectionné dans le groupe constitué par le magnésium, le calcium, le strontium et le baryum, de préférence par le magnésium et le calcium, de manière très préférée par le magnésium.

Le métal alcalin, lorsqu'il est présent, est réparti de manière homogène dans le support avec un coefficient R (décrit par la suite) compris entre 0,8 et 1,2.

Le métal alcalino-terreux, lorsqu'il est présent, est réparti de manière homogène dans le support avec un coefficient R (décrit par la suite) compris entre 0,8 et 1,2.

Le support poreux est choisi parmi les oxydes de magnésium, d'aluminium, de silicium, de zirconium, de thorium, de titane, ou de cérium, pris seuls ou en mélange entre eux. De préférence, le support est un oxyde d'aluminium (alumine) ou de silicium (silice). De façon encore plus préférée, le support est de l'alumine. L'alumine peut être présente sous toutes les formes cristallographiques possibles : alpha, delta, téta, chi, gamma, etc., prises seules ou en mélange. De manière préférée le support est choisi parmi l'alumine alpha, delta, téta. De manière très préférée on choisit l'alumine alpha.

La surface spécifique du support poreux est comprise entre 1 et 300 m$^2$/g, de préférence entre 2 et 200 m$^2$/g, et encore plus préférentiellement entre 3 et 150 m$^2$/g. La surface spécifique BET est mesurée par physisorption à l'azote. La surface spécifique BET est mesurée par physisorption à l'azote selon la norme ASTM D3663-03 tel que décrit dans Rouquerol F.; Rouquerol J.; Singh K. « Adsorption by Powders & Porous Solids: Principle, methodology and applications », Academic Press, 1999.

Le volume total poreux du support est compris entre 0,1 et 1,5 cm$^3$/g, de préférence compris entre 0,2 et 1,4 cm$^3$/g, et encore plus préférentiellement compris entre 0,25 et 1,3 cm$^3$/g. Le volume poreux total est mesuré par porosimétrie au mercure selon la norme ASTM D4284-92 avec un angle de mouillage de 140°, par exemple au moyen d'un appareil modèle Autopore® III de la marque Microméritics®.

Selon l'invention, le grain de support poreux se présente sous forme de billes, de trilobes, d'extrudés, de pastilles, ou d'agglomérats irréguliers et non sphériques dont la forme spécifique peut résulter d'une étape de concassage. Ledit grain de support se présente sous forme de billes ou d'extrudés. De manière encore plus avantageuse, ledit grain de support se présente sous forme de billes. La taille des grains est entre 1 mm et 10 mm, de préférence entre 2 et 8 mm.

Le catalyseur de la présente invention peut être caractérisé par plusieurs paramètres qui seront décrits par la suite, notamment :

- le coefficient R qui exprime la répartition homogène de l'or dans un grain de support poreux ;
- l'épaisseur de la croûte de palladium formée à la périphérie du grain de support poreux ;
- la dispersion métallique D qui permet de déduire la taille moyenne des particules métalliques d'or.

## Dispersion métallique D des particules

La dispersion de particules est un nombre sans unité, souvent exprimé en %. La dispersion est d'autant plus grande que les particules sont petites. Elle est définie dans publication de R. Van Hardeveld et F. Hartog, «The statistics of surface atoms and surface sites on metal crystals», Surface Science 15, 1969, 189-230.

## Définition du coefficient R

Les profils de répartition des éléments au sein des grains de catalyseurs sont obtenus par microsonde de Castaing. Au moins 30 points d'analyses sont effectués le long du diamètre de la bille ou de l'extrudé à raison d'une dizaine de points sur la croûte d'un élément actif (ici l'or) et d'une dizaine de point au centre du grain. Il est ainsi obtenu le profil de répartition $c(x)$ pour $x \in [-r;+r]$ avec c la concentration locale de l'élément, r le rayon de la bille ou de l'extrudé et x la position du point d'analyse le long du diamètre du grain par rapport au centre de ce grain.

La répartition des éléments est caractérisée par un coefficient R adimensionnel pondérant la concentration locale par un poids croissant en fonction de la position sur le diamètre. Par définition :

$$ R = \int_{-r}^{r} c(x)x^2 dx \bigg/ \frac{r^2}{3} \int_{-r}^{r} c(x)dx $$

Ainsi un élément dont la concentration est uniforme présente un coefficient R égal à 1, un élément déposé en dôme (concentration au cœur plus importante que la concentration aux bords du support) présente un coefficient supérieur à 1 et un élément réparti en croûte (concentration aux bords plus importante que la concentration au coeur du support) présente un coefficient inférieur à 1. L'analyse par microsonde de Castaing donne les valeurs des concentrations en un nombre fini de valeurs de x, R est ainsi évalué numériquement par des méthodes d'intégration bien connues de l'homme du métier. De préférence, R est déterminé par la méthode des trapèzes.

La répartition de l'or est définie comme homogène lorsque le coefficient de répartition R défini ci-dessus est compris entre 0,8 et 1,2.

La répartition de l'élément alcalin est définie comme homogène lorsque le coefficient de répartition R défini ci-dessus est compris entre 0,8 et 1,2.

La répartition de l'élément alcalino-terreux est définie comme homogène lorsque le coefficient de répartition R défini ci-dessus est compris entre 0,8 et 1,2.

## Définition de l'épaisseur de croûte de palladium

Afin d'analyser la répartition de la phase métallique sur le support, on mesure une épaisseur de croûte par microsonde de Castaing (ou microanalyse par microsonde électronique). L'appareil utilisé est un CAMECA XS100, équipé de quatre cristaux monochromateurs permettant l'analyse simultanée de quatre éléments. La technique d'analyse par microsonde de Castaing consiste en la détection de rayonnement X émis par un solide après excitation de ses éléments par un faisceau d'électrons de hautes énergies. Pour les besoins de cette caractérisation, les billes de catalyseur sont enrobées dans des plots de résine époxy. Ces plots sont polis jusqu'à atteindre la coupe au diamètre des billes puis métallisés par dépôt de carbone en évaporateur métallique. La sonde électronique est balayée le long du diamètre de cinq billes pour obtenir le profil de répartition moyen des éléments constitutifs des solides.

Lorsque le palladium est reparti en croûte, sa concentration locale diminue généralement progressivement lorsqu'elle est mesurée en partant du bord du grain catalytique vers l'intérieur. Une distance du bord du grain, à laquelle la teneur locale en palladium devient nulle, ne peut souvent pas être déterminée avec précision et reproductibilité. Afin de mesurer une épaisseur de croûte qui est significative pour la majorité des particules de palladium, l'épaisseur de croûte est définie comme la distance au bord du grain contenant 80 % en poids de palladium.

Pour mesurer une épaisseur de croûte qui est significative pour la majorité des particules de palladium, l'épaisseur de croûte peut de façon alternative être définie comme la distance au bord du grain contenant 80% en poids du palladium. A partir du profil de répartition obtenu par la microsonde de Castaing (c(x)), on peut calculer la quantité cumulée $Q(y)$ de palladium dans le grain en fonction de la distance y au bord du grain de rayon r.

Pour une bille :

$$Q(y) = \int_{-r}^{-y} c(x) 4\pi.x^2 dx + \int_{y}^{r} c(x) 4\pi.x^2 dx$$

Pour un extrudé :

$$Q(y) = \int_{-r}^{-y} c(x) 2\pi.x dx + \int_{y}^{r} c(x) 2\pi.x dx$$

$Q(r)$ correspond ainsi à la quantité totale de l'élément dans le grain. On résout ensuite numériquement l'équation suivante en y :

$$\frac{Q(y)}{Q(r)} = 0,8$$

c étant une fonction strictement positive, Q est donc une fonction strictement croissante et cette équation possède une seule solution qui est l'épaisseur de croûte.

Procédé de préparation du catalyseur

Le procédé de préparation du catalyseur comprend d'une manière générale les étapes suivantes :

- une première étape, dite étape 1, dans laquelle on introduit le palladium sur au moins un grain de support poreux, le palladium étant déposé par toute méthode connue de l'homme du métier, de manière à obtenir une croûte inférieure à 300 $\mu$m, suivie d'un séchage et d'une calcination ;
- une deuxième étape, dite étape 2, dans laquelle on introduit l'or.

Il est important de souligner que le procédé de préparation est effectué en deux étapes distinctes incluant une étape de calcination intermédiaire entre les deux étapes de dépôts de métal.

Les étapes 1 et 2 peuvent être effectuées dans un ordre quelconque. De préférence, l'étape 1 est réalisée avant l'étape 2. Si l'étape 2 est effectuée avant l'étape 1, l'étape de réduction 2f) est nécessaire.

L'étape 1) consiste en la préparation d'un support poreux imprégné par imprégnation de palladium sur au moins un grain de support poreux de manière à ce que le palladium soit réparti dans une croûte à la périphérie dudit grain de support, l'épaisseur de ladite croute étant inférieure à 300 $\mu$m. Le dépôt de la solution de palladium sur le support peut être réalisé selon toutes les techniques connues de l'homme du métier. De préférence, la solution de palladium est déposée par méthode colloïdale.

L'étape 1) comprend les étapes suivantes :

1a) on prépare une solution contenant un précurseur de palladium, de préférence une suspension colloïdale, d'oxyde de palladium ou d'hydroxyde de palladium en phase aqueuse ;

1b) on imprègne ladite solution sur au moins un grain de support poreux ;

1c) on soumet le support poreux imprégné obtenu à l'étape 1b) à une maturation afin d'obtenir un précurseur de catalyseur ;

1d) on sèche le précurseur de catalyseur obtenu à l'étape 1c) à une température comprise entre 50°C et 250°C ;

1e) on calcine le précurseur de catalyseur obtenu à l'étape 1d) à une température comprise entre 250°C et 900°C.

L'étape 2) comprend les étapes suivantes :

2a) on prépare une solution aqueuse contenant un précurseur d'or ;

2b) on imprègne ledit support poreux avec la ladite solution obtenue à l'étape 2a);

2c) on soumet le support poreux imprégné obtenu à l'étape 2b) à une maturation afin d'obtenir un précurseur de catalyseur ;

2d) on met en contact le précurseur de catalyseur obtenu à l'étape 2c) avec une solution contenant de l'urée ;

2e) on sèche le précurseur de catalyseur obtenu à l'étape 2d) à une température comprise entre 50°C et 300°C, sous air ;

2f) optionnellement, on soumet le catalyseur séché obtenu à l'issue de l'étape 2e) à un traitement réducteur par mise en contact avec un gaz réducteur.

Selon l'invention, l'étape 2) ne comprend pas de traitement thermique oxydant ou neutre, non réducteur, du précurseur de catalyseur afin d'éviter la formation de grosses particules d'or (i.e. supérieure à 5 nm).

Les différentes étapes 1 et 2 sont par la suite détaillées.

Une étape optionnelle de séchage du précurseur obtenu à l'étape 2c) peut être réalisée par séchage dudit précurseur à une température comprise entre 50°C et 300°C.

Une étape optionnelle de dépôt d'un métal appartenant au groupe IB (autre que l'or), de préférence l'argent ou le cuivre, peut être également réalisée, entre les étapes 1 et 2 ou simultanément à l'étape 1.

Le catalyseur peut comprendre au moins un métal sélectionné dans le groupe constitué par les alcalins et les alcalino-terreux. L'ajout d'alcalin/ alcalino-terreux peut être introduit d'une manière séparée avant, pendant ou après toute étape de dépôt du palladium, éventuellement après dépôt du métal appartenant au groupe IB (autre que l'or) .

L'étape 2 est toujours précédée un traitement thermique oxydant (calcination). Il n'y a pas de calcination après l'étape 2.

## Étape 1 : Dépôt du palladium

### Étape 1a) préparation d'une suspension colloïdale d'oxyde de palladium ou d'hydroxyde de palladium en phase aqueuse

La suspension colloïdale est généralement obtenue par hydrolyse du cation palladium en milieu aqueux, ce qui conduit à la formation de particules d'oxyde ou d'hydroxyde de palladium en suspension.

La solution aqueuse d'hydroxyde d'alcalins ou d'hydroxyde alcalino-terreux est généralement sélectionnée dans le groupe constitué par les solutions aqueuses d'hydroxyde de sodium, les solutions aqueuses d'hydroxyde de magnésium. De manière préférée, de préférence la solution aqueuse est une solution aqueuse d'hydroxyde de sodium.

Le sel précurseur du palladium est généralement sélectionné dans le groupe constitué par le chlorure de palladium, le nitrate de palladium et le sulfate de palladium. De manière très préférée, le sel précurseur du palladium est le nitrate de palladium.

Typiquement, on approvisionne dans un appareillage adapté la solution aqueuse comprenant au moins un sel précurseur du palladium [appelée aussi ici solution (II)] puis la solution aqueuse comprenant au moins un hydroxyde d'alcalins ou d'alcalino-terreux [appelée aussi ici solution (I)]. Alternativement, les solutions (I) et (II) peuvent être versées simultanément dans l'appareillage. De préférence, la solution aqueuse (II) puis la solution aqueuse (I) est versée dans l'appareillage.

La suspension colloïdale reste généralement dans l'appareillage pendant un temps de séjour compris entre 0 et 20 heures.

Les concentrations de la solution (I) et (II) sont généralement choisies afin d'obtenir un pH de la suspension colloïdale compris entre 1,0 et 3,5. Ainsi, le pH de la suspension colloïdale peut être modifié pendant ce temps de séjour par ajout de quantités d'acide ou de base compatibles avec la stabilité de la suspension colloïdale.

En général, la température de préparation est comprise entre 5°C et 40°C et de manière préférée entre 15°C et 35°C.

La concentration en palladium est de préférence comprise entre 5 et 150 millimoles par litre (mmol/L), de manière plus préférée entre 8 et 80 millimoles par litre.

Étape 1b) dépôt de la suspension colloïdale préparée à l'étape 1a) par imprégnation sur un support, de préférence sur de l'alumine

La suspension colloïdale préparée à l'étape 1a) est ensuite imprégnée sur un support.

Le support peut éventuellement subir un ensemble de traitements avant l'étape d'imprégnation, tel que des calcinations ou des hydratations. Le support peut aussi déjà comprendre un ou plusieurs éléments métalliques avant l'imprégnation de la suspension colloïdale. Des éléments métalliques peuvent aussi être introduits dans la suspension colloïdale. Ces éléments métalliques peuvent être introduits soit par des techniques conventionnelles, soit en utilisant le procédé selon la présente invention.

La suspension colloïdale est de préférence versée sur le support. Ce processus peut être réalisé soit de façon discontinue, c'est-à-dire que l'étape de préparation de la suspension colloïdale précède l'étape d'imprégnation sur le support et que l'essentiel de la suspension colloïdale est envoyée en une seule fois vers l'étape d'imprégnation, soit en continu, c'est-à-dire que le produit obtenu dans l'étape 1a) est envoyé en continu après ajustement du temps de séjour de la suspension colloïdale à l'étape 1b).

On peut par exemple citer comme processus continu, un processus où les solutions I et II sont versées simultanément dans un bac qui se déverse en continu dans une zone comprenant le support à imprégner.

Étape 1c) maturation du support imprégné lors de l'étape b) pendant une durée comprise entre 0,5 et 40 heures

Après imprégnation, le support poreux imprégné est maturé pendant 0,5 à 40 heures, de manière préférée pendant 1 à 30 heures, de préférence à température ambiante.

De préférence, ladite étape de maturation est effectuée sous air et de préférence sous air humide avec une humidité relative entre 20 et 100% et de préférence entre 70 et 100%.

Étape 1d) séchage du précurseur de catalyseur obtenu à l'étape c)

Le précurseur du catalyseur est généralement séché afin d'éliminer toute ou une partie de l'eau introduite lors de l'imprégnation, de préférence à une température comprise entre 50°C et 250°C, de manière plus préférée entre 70°C et 200°C. La durée du séchage est de préférence comprise entre 0,5 et 20 heures.

Le séchage est généralement effectué sous air de combustion d'un hydrocarbure, de préférence du méthane, ou sous air chauffé comprenant entre 0 et 80 grammes d'eau par kilogramme d'air de combustion, un taux d'oxygène compris entre 5% et 25% volume et un taux de dioxyde de carbone compris entre 0% et 10% volume.

Étape 1e) calcination sous air de combustion du catalyseur séché obtenu à l'étape 1d)

Après séchage, le catalyseur est généralement calciné sous air de combustion, de préférence un air de combustion du méthane comprenant entre 40 et 80 grammes d'eau par kg d'air de combustion, un taux d'oxygène compris entre 5% et 15% volume et un taux de $CO_2$ compris entre 4% et 10% volume. La température de calcination est comprise entre 250°C et 900°C, de préférence comprise entre environ 300°C et environ 500°C. La durée de calcination est généralement comprise entre 0,5 et 5 heures.

Selon une variante, le catalyseur peut contenir un ou plusieurs métaux promoteurs. Le ou les métaux promoteurs peuvent être introduit lors de la préparation du support, sur le support déjà formé, pendant l'étape 1a) ou à l'issue des étapes 1b), 1c), 1d), ou 1e).

**Etape 2 : Dépôt d'or**

Étape 2a) préparation d'une solution d'imprégnation en phase aqueuse

La solution est préparée par dissolution d'un sel précurseur d'or.

Le sel du précurseur d'or utilisé présente un degré d'oxydation du métal supérieur à 0 et est soluble en solution aqueuse. Le sel du précurseur d'or peut être par exemple un halogénure. Il peut être choisi de façon préférée dans le groupe constitué par les chlorures d'or comme le trichlorure d'or, l'acide tetrachloroaurique, le tetrachloraurate de sodium ou de potassium. De préférence le précurseur utilisé est l'acide tetrachloroaurique.

En général, la température de préparation est comprise entre 5°C et 40°C et de manière préférée entre 15°C et 35°C. La concentration en or de la solution est de préférence comprise entre 1 mmol/L et 1 mol/L, soit 0,2g/L à 200g/L.

### Étape 2b) dépôt de la solution préparée à l'étape 2a)

La solution préparée à l'étape 2a) est ensuite imprégnée sur le support poreux. L'imprégnation du support peut être réalisée par imprégnation à sec, en excès ou en défaut, en mode statique ou dynamique. L'imprégnation à sec est préférée. L'imprégnation peut être réalisée en une ou plusieurs imprégnations successives.

L'étape d'imprégnation à sec consiste à mettre en contact ledit support poreux avec au moins une solution, contenant au moins un précurseur d'or, dont le volume est égal au volume poreux dudit support à imprégner. Cette solution contient du précurseur métallique d'or à la concentration voulue pour obtenir sur le catalyseur final la teneur en or visée.

Le support poreux comprend une surface spécifique comprise entre 1 et 300 m$^2$/g, de préférence entre 2 à 200 m$^2$/g, et encore plus préférentiellement entre 3 à 150 m$^2$/g.

Le support peut éventuellement subir un ensemble de traitements avant l'étape d'imprégnation, tel que des calcinations ou des hydratations.

### Étape 2c) maturation du support imprégné obtenu à l'étape 2b)

Après imprégnation, le support poreux imprégné est maturé pendant 0,5 à 40 heures, de manière préférée pendant 1 à 30 heures, de préférence à température ambiante. De préférence, ladite étape de maturation est effectuée sous air et de préférence sous air humide avec une humidité relative entre 20 et 100% et de préférence entre 70 et 100%.

De manière optionnelle, le précurseur du catalyseur peut être séché afin d'éliminer toute ou une partie de l'eau introduite lors de l'imprégnation, de préférence à une température comprise entre 50°C et 300°C, de manière plus préférée entre 70°C et 250°C. La durée du séchage est comprise entre 0,5 h et 20 h. Le séchage est généralement effectué sous air de combustion d'un hydrocarbure, de préférence du méthane, ou sous air chauffé comprenant entre 0 et 80 grammes d'eau par kilogramme d'air de combustion, un taux d'oxygène compris entre 5% et 25% volume et un taux de dioxyde de carbone compris entre 0% et 10% volume.

### Étape 2d) traitement avec de l'urée

Le catalyseur est mis en contact, de préférence sous agitation, avec une solution aqueuse comprenant au moins de l'urée.

Le volume de la solution aqueuse contenant au moins de l'urée est compris entre 0,9 et 20 fois le volume poreux du catalyseur mis en forme.

Le rapport molaire urée/or est compris entre 1 et 1000, de préférence entre 2 et 700, de manière très préférée entre 3 et 300.

En général, la température de la solution est maintenue constante et est comprise entre 5°C et 120°C, et de manière préférée entre 15°C et 100°C. Le temps de séjour de ladite solution aqueuse dans l'appareillage est compris entre 0,5 et 20 heures.

L'urée est diluée dans un solvant organique, par exemple de l'éthanol, et/ou aqueux, de préférence le solvant est de l'eau.

La solution contenant de l'urée peut également contenir d'autres composés organiques comme de l'ammoniaque De préférence, la solution contient uniquement de l'urée et de l'eau.

### Filtration / Lavage

Optionnellement, le précurseur de catalyseur obtenu à l'étape d) peut être filtré selon toute technique connue de l'homme du métier.

Optionnellement, le précurseur de catalyseur est lavé, de préférence avec de l'eau. Le volume total d'eau engagé pour le(s) étape(s) de lavage est compris entre 1 et 30 fois le volume catalytique engagé. Le lavage peut être réalisé en une ou plusieurs étapes. Le lavage du précurseur de catalyseur permet d'éliminer la présence d'urée et de chlore dans le précurseur de catalyseur.

La durée du lavage est généralement comprise entre 1 minute et 10 heures, de préférence entre 5 minutes et 8 heures, et encore plus préférentiellement entre 10 minutes et 7 heures Le lavage permet de diminuer la quantité d'élément chlore éventuellement présent dans la précurseur de catalyseur.

### Étape 2e) Séchage du précurseur de catalyseur obtenu à l'étape 2d)

Le catalyseur est généralement séché afin d'éliminer toute ou une partie de l'eau introduite lors de l'imprégnation, de préférence à une température comprise entre 50 et 300°C, de manière plus préférée entre 70°C et 250°C. La durée du séchage est comprise entre 0,5 et 20 heures.

Le séchage est généralement effectué sous air de combustion d'un hydrocarbure, de préférence du méthane, ou sous air chauffé comprenant entre 0 et 80 grammes d'eau par kilogramme d'air de combustion, un taux d'oxygène compris entre 5% et 25% volume et un taux de dioxyde de carbone compris entre 0% et 10% volume.

Étape 2f) Traitement thermique sous atmosphère réductrice du catalyseur séché obtenu à l'étape 2e)

Optionnellement, après séchage, le catalyseur est réduit. Cette étape est de préférence réalisée en présence d'un gaz réducteur, soit in-situ, c'est-à-dire dans le réacteur où est réalisée la transformation catalytique, soit ex-situ. La température de réduction est généralement comprise entre 40°C et 500°C, de préférence comprise entre environ 100°C et environ 400°C.

La réduction est réalisée en présence d'un gaz réducteur comprenant entre 25 vol% et 100 vol% d'hydrogène, de préférence 100% volume d'hydrogène. L'hydrogène est éventuellement complété par un gaz inerte pour la réduction, de préférence de l'argon, de l'azote ou du méthane. La réduction comprend généralement une phase de montée en température puis un palier.

La durée du palier de réduction est généralement comprise entre 0,5 et 10 heures, de préférence entre 2 et 8 heures.

La VVH est généralement comprise entre 150 et 3000, de préférence entre 300 et 1500 litres de gaz réducteur par heure et par litre de catalyseur. Par "V.V.H." on entend au sens de la présente invention, la vitesse volumétrique horaire définit comme le rapport entre le débit volumique de la charge à traité et le volume de catalyseur chargé dans le réacteur. La vitesse volumétrique horaire s'exprime en $h^{-1}$.

Dans le mode de réalisation pour lequel l'étape 2 est réalisée avant l'étape 1, l'étape 2f) est nécessaire.

Utilisation du catalyseur selon l'invention

Le catalyseur selon l'invention peut être utilisé dans les réactions d'hydrogénation des composés comportant des fonctions acétyléniques, diéniques, oléfiniques.

L'invention concerne en particulier la mise en contact du catalyseur selon l'invention avec une charge étant sélectionnée dans le groupe constitué par les coupes C2, C3, les coupes C4, les coupes C5 de vapocraquage et/ou de craquage catalytique et les essences de vapocraquage appelée aussi essences de pyrolyse, de manière préférée les charges sont des coupes C2, C3, C4 de vapocraquage et/ou de craquage catalytique.

En fonction de l'agencement des unités en aval du vapocraqueur, les charges étudiées pour l'hydrogénation sélective auront des compositions variables. La nature et la teneur en hydrocarbures, le rapport $H_2$ / HC, la nature et la teneur des impuretés (soufre, CO, $CO_2$) varient selon le type de configuration.

L'hydrogénation sélective en tête de réacteur (appelée aussi hydrogénation sélective en configuration « Front end » selon la terminologie anglo-saxonne) est généralement opérée en phase gaz. La composition typique de charges d'hydrogénation sélective en configuration « Font end » comprend une teneur en acétylène comprise entre 0,15% et 0,5%, une teneur en éthylène 20% et 50%, une teneur en éthane entre 5% et 15% et une teneur en hydrogène entre 5 et 20%, une teneur en méthane entre 20 et 40%, une teneur en CO comprise entre 50 et 3000ppm mol. Il existe donc un très large excès d'hydrogène par rapport à l'acétylène dans les charges en conditions « Front end ». Les teneurs en CO sont variables. Il est connu que le CO s'adsorbe sur les sites actifs métalliques du catalyseur diminuant son activité. Une diminution soudaine de CO va libérer des sites pour l'hydrogénation et donc conduire à une suractivité et potentiellement à un emballement du réacteur. A l'opposé, une augmentation de la teneur en CO conduit à une baisse des performances du catalyseur. De manière très préférée le catalyseur est mis en contact avec une charge en conditions « Front end » de coupe C2 ou C2/C3.

Les conditions opératoires préférées de l'hydrogénation sélective en conditions « Front end » sont les suivantes : une température comprise entre 30 et 200°C, une pression entre 1,5 et 4,0 MPa et une vitesse spatiale entre 1 000 $h^{-1}$ et 30 000 $h^{-1}$.

Exemples

Les exemples présentés ci-dessous visent à démontrer l'amélioration de l'activité catalytique en hydrogénation sélective. Les exemples 1 à 4 et 7 et 8 concernent des procédés de préparation de catalyseurs non conformes à l'invention et les exemples 5 et 6 concernent un procédé de préparation d'un catalyseur selon l'invention.

L'exemple 9 concerne l'application de ces catalyseurs dans une réaction d'hydrogénation sélective. Ces exemples sont présentés à titre d'illustration et ne limitent en aucune manière la portée de l'invention.

Exemple 1 : préparation d'un catalyseur C1 : 0,06% poids Pd / δ-Al$_2$O$_3$ (non conforme)

Cet exemple montre la préparation d'un catalyseur palladium sur alumine delta.

Une suspension colloïdale d'oxyde de palladium est préparée sous agitation à 25°C par dilution de 0,60 gramme d'une solution de nitrate de palladium Pd(NO$_3$)$_2$ contenant 8,5 % poids de palladium avec 45 mL d'eau déminéralisée, puis ajout d'environ 10 mL d'une solution de soude. La suspension est ensuite diluée avec de l'eau déminéralisée à un volume qui correspond au volume poreux du support alumine. Cette solution est ensuite imprégnée sur 80 grammes d'une alumine dont la surface spécifique est de 140 m$^2$/g, mise en forme sous forme de billes 2-4 mm. Une étape de maturation du support imprégné avant séchage d'une durée de 20 heures est effectuée sous air en milieu confiné et humide. Le solide obtenu est séché sous air pendant 2 heures à 120°C. Le catalyseur est ensuite calciné sous un flux d'air 2 heures à 450°C.

Le catalyseur C1 contient 0,06 % poids de palladium par rapport au poids total du catalyseur. La caractérisation du catalyseur C1 par microsonde de Castaing montre que 80% du palladium est réparti sur une croûte d'épaisseur inférieure à 250 μm.

Exemple 2 : préparation d'un catalyseur C2 : 0,06% poids Pd / α-Al$_2$O$_3$ (non conforme)

Cet exemple montre la préparation d'un catalyseur palladium sur alumine alpha.

Une suspension colloïdale d'oxyde de Pd est préparée sous agitation à 25°C par dilution de 0,60 gramme d'une solution de nitrate de palladium Pd(NO$_3$)$_2$ contenant 8,5 % poids de palladium avec environ 45 mL d'eau déminéralisée, puis ajout d'environ 10 mL d'une solution de soude. La suspension est ensuite diluée avec de l'eau déminéralisée à un volume qui correspond au volume poreux du support alumine. Cette solution est ensuite imprégnée sur 80g d'une alumine dont la surface spécifique est de 10 m$^2$/g mise en forme sous forme de billes 2-4 mm. Une étape de maturation du support imprégné avant séchage d'une durée de 20 h est effectuée sous air en milieu confiné et humide. Le solide obtenu est séché sous air pendant 2 h à 120°C. Le catalyseur est ensuite calciné sous un flux d'air 2 h à 450°C.

Le catalyseur C2 contient 0,06 % poids de palladium par rapport au poids total du catalyseur. La caractérisation du catalyseur C2 par microsonde de Castaing montre que 80% du Pd est réparti sur une croûte d'épaisseur inférieure à 250 μm.

Exemple 3 : préparation d'un catalyseur C3 : Pd (0.06% poids) + Au (2% poids) / δ-Al$_2$O$_3$ (non-conforme)

Cet exemple montre la préparation classique d'un catalyseur comprenant du palladium et de l'or par imprégnation à sec seulement (et donc sans lavage à l'urée).

Une solution mère de concentration 20 g/L (102 mmol/L) d'or est préparée par dilution avec de l'eau sous agitation à 25°C de deux grammes d'HAuCl$_4$.3H$_2$O avec environ 50 mL d'eau déminéralisée. Une partie de la suspension est ensuite imprégnée sur 20 grammes de catalyseur C1. Une étape de maturation d'une durée de 20 h du support imprégné avant séchage est effectuée sous air en milieu confiné et humide. Le solide obtenu est séché sous air 2 h à 100°C. Le catalyseur est ensuite réduit sous un flux d'hydrogène 2 h à 300°C.

Le catalyseur C3 contient 2% poids d'or, 1,4% poids de chlore et 0,06% poids de palladium par rapport au poids total du catalyseur. Le rapport massique Au / Pd du catalyseur est de 33.

La caractérisation du catalyseur par microsonde de Castaing montre que l'or est réparti de façon homogène avec un coefficient de répartition R (Au) = 0,91.

La caractérisation du catalyseur par microsonde de Castaing montre que le chlore est réparti de façon homogène avec un coefficient de répartition R(Cl) = 0,89.

La taille moyenne des particules d'or mesurée par microscopie électronique à transmission est de 30 nm. La dispersion moyenne correspondante est de 4%.

La caractérisation du catalyseur C3 par microsonde de Castaing montre que 80% du palladium est réparti sur une croûte d'épaisseur inférieure à 250 μm.

Exemple 4 : préparation d'un catalyseur C4 : Pd (0,06% poids) + Au (2% poids) / δ-Al$_2$O$_3$ (non-conforme)

Cet exemple montre la préparation d'un catalyseur dont l'or est introduit dans le catalyseur par dépôt - précipitation et dans laquelle l'or et l'urée sont introduits simultanément, en solution, telle que décrit par exemple dans le document FR 2932177.

Une suspension contenant 20 grammes de catalyseur C1 mis en forme sous forme de billes et 150 mL d'eau sont placés dans un réacteur et chauffés à 80°C. 20 mL de solution à 20g/L (102 mmol/L) d'or sont introduits dans le réacteur. 12 grammes d'urée dilués dans 20mL d'eau déminéralisée sont alors ajoutés. Le rapport molaire urée / or est de 100. La suspension est agitée pendant 6 heures. Le solide est filtré sur Buchner puis lavé trois fois avec 150 mL d'eau.

Le solide obtenu est séché sous air 2 heures à 100°C. Le catalyseur est ensuite réduit sous un flux d'hydrogène 2 heures à 300°C.

Le catalyseur C4 contient 1,7 % poids d'or, une teneur en chlore inférieure à 300 ppm poids par rapport au poids total du catalyseur, et 0,06% poids de palladium par rapport au poids total du catalyseur. Le rapport massique Au / Pd du catalyseur est de 28.

La caractérisation du catalyseur C4 par microsonde de Castaing montre que l'or est réparti en périphérie du catalyseur avec un coefficient de répartition R (Au) = 0,49.

La taille moyenne des particules d'or mesurée par microscopie électronique à transmission est de 4 nm.

La dispersion moyenne correspondante est de 33%.

La caractérisation du catalyseur C4 par microsonde de Castaing montre que 80% du palladium est réparti sur une croûte d'épaisseur inférieure à 250 $\mu$m.

Exemple 5: préparation d'un catalyseur C5 conforme à l'invention

Une solution mère de concentration 20 g/L (102 mmol/L) d'or est préparée par dilution avec de l'eau sous agitation à 25°C de 2 grammes d'HAuCl$_4$.3H$_2$O avec environ 50 mL d'eau déminéralisée. Une partie de la solution est ensuite imprégnée sur 20 grammes de catalyseur C1 mise en forme sous forme de billes 2-4 mm. Une étape de maturation d'une durée de 20 heures du support imprégné avant séchage est effectuée sous air en milieu confiné et humide. Le solide obtenu est séché sous air 2 heures à 100°C.

Le catalyseur est ensuite imprégné au volume poreux d'une solution d'urée de concentration 80 g/L. La suspension est agitée à 70°C pendant 4 heures. Le rapport molaire urée / or est de 13. Le solide est ensuite filtré et lavé 4 fois avec 150 mL d'eau. Le solide obtenu est séché sous air 2 heures à 120°C. Le catalyseur est ensuite réduit sous un flux d'hydrogène 2 heures à 300°C.

Le catalyseur C5 contient 1,9% poids d'or, une teneur en chlore inférieure à 0,03% poids par rapport au poids total du catalyseur et 0,06% poids de palladium par rapport au poids total du catalyseur. Le rapport massique Au / Pd du catalyseur est de 32.

La caractérisation du catalyseur par microsonde de Castaing montre que l'or est réparti de façon homogène avec un coefficient de répartition R (Au) = 1,12.

La taille moyenne des particules d'or mesurée par microscopie électronique à transmission est de 2,6 nm. La dispersion moyenne correspondante est de 44%.

Le catalyseur C5 contient 0,06% poids de palladium. La caractérisation du catalyseur C5 par microsonde de Castaing montre que 80% du Pd est réparti sur une croûte d'épaisseur inférieure à 250 $\mu$m.

Exemple 6: préparation d'un catalyseur C6 conforme à l'invention

Une solution mère de concentration 30 g/L (153 mmol/L) d'or est préparée par dilution avec de l'eau sous agitation à 25°C de 2 grammes d'HAuCl$_4$.3 H$_2$O avec environ 35 mL d'eau déminéralisée. La solution est imprégnée sur 20 grammes de catalyseur C2 mise en forme sous forme de billes. Une étape de maturation d'une durée de 20 heures du support imprégné avant séchage est effectuée sous air en milieu confiné et humide. Le solide obtenu est séché sous air 2 heures à 100°C.

Le catalyseur est ensuite imprégné d'une solution d'urée de concentration 100 g/L. La suspension est agitée à 70°C pendant 4 heures. Le rapport molaire urée / or est de 12.

Le solide est ensuite filtré et lavé 4 fois avec 150 mL d'eau. Le catalyseur est ensuite réduit sous un flux d'hydrogène 2 heures à 300°C.

Le catalyseur C6 contient 1,7 % poids d'or, une teneur en chlore inférieure à 0,03 % poids par rapport au poids total du catalyseur et 0,06% poids de palladium par rapport au poids total du catalyseur. Le rapport massique Au / Pd du catalyseur est de 26.

La caractérisation du catalyseur par microsonde de Castaing montre que l'or est réparti de façon homogène avec un coefficient de répartition R (Au) = 0,95.

La taille moyenne des particules d'or mesurée par microscopie électronique à transmission est de 3,1 nm. La dispersion moyenne correspondante est de 40%.

Le catalyseur C6 contient 0,06 % poids de palladium. La caractérisation du catalyseur C6 par microsonde de Castaing montre que 80% du palladium est réparti sur une croûte d'épaisseur inférieure à 250 $\mu$m.

Exemple 7 : préparation d'un catalyseur C7 : 0,02% poids Pd / $\alpha$-Al$_2$O$_3$ (non conforme)

Cet exemple montre une méthode de préparation d'un catalyseur comprenant du palladium sur alumine alpha.

Une suspension de précurseur de sel de Pd est préparée sous agitation à 25°C par dilution de 0,20 grammes d'une solution de nitrate de palladium Pd(NO$_3$)$_2$ contenant 8,5 % poids de palladium avec environ 45 mL d'eau déminéralisée. La suspension est ensuite diluée avec de l'eau déminéralisée à un volume qui correspond au volume poreux du support alumine. Cette solution est ensuite imprégnée sur 80g d'une alumine alpha dont la surface spécifique est de 10 m$^2$/g mise en forme sous forme de billes 2-4 mm. Le solide obtenu est séché sous air pendant 2 h à 120°C. Le catalyseur est ensuite calciné sous un flux d'air 2 h à 450°C.

Le catalyseur C7 contient 0,02 % poids de palladium par rapport au poids total du catalyseur. La caractérisation du catalyseur C2 par microsonde de Castaing montre que 80% du Pd est réparti sur une croûte d'épaisseur inférieure à 250 $\mu$m.

Exemple 8 : préparation d'un catalyseur C8 : Pd (0,02% poids) + Au (0,8% poids) / $\alpha$ -Al$_2$O$_3$ (non-conforme)

Cet exemple montre la préparation d'un catalyseur comprenant du palladium et de l'or par imprégnation à sec (mais sans lavage à l'urée), telle que décrit par exemple dans le document US 6,509,292.

Une solution mère de concentration 20 g/L (102 mmol/L) d'or est préparée par dilution avec de l'eau sous agitation à 25°C d'un gramme d'HAuCl$_4$.3 H$_2$O avec environ 25 mL d'eau déminéralisée. Une partie de la solution est ensuite imprégnée sur 20 grammes de catalyseur C7 mis en forme sous forme de billes. Le solide obtenu est calciné sous air 3 heures à 450°C.

Le catalyseur est ensuite réduit en phase aqueuse en présence d'une solution de formiate de sodium à 70°C pendant 1h. Le catalyseur est ensuite lavé avec de l'eau à 70 °C puis séché une nuit à 120°C.

Le catalyseur C8 contient 0,8 % poids d'or, une teneur en chlore inférieure à 0,01 % poids par rapport au poids total du catalyseur et 0,02% poids de palladium par rapport au poids total du catalyseur. Le rapport massique Au / Pd du catalyseur est de 40.

La caractérisation du catalyseur par microsonde de Castaing montre que l'or est réparti de avec un coefficient de répartition R (Au) = 0,90

La taille moyenne des particules d'or mesurée par microscopie électronique à transmission est de 30 nm. La dispersion moyenne correspondante est de 4%.

Le catalyseur C8 contient 0,02 % poids de palladium. La caractérisation du catalyseur C8 par microsonde de Castaing montre que 80% du palladium est réparti sur une croûte d'épaisseur inférieure à 250 $\mu$m.

Le tableau 1 suivant résume les caractéristiques des différents catalyseurs C1 à C8

Tableau 1

| Catalyseur | C1 | C2 | C3 | C4 | C5 | C6 | C7 | C8 |
|---|---|---|---|---|---|---|---|---|
| Support | 5-Al$_2$O$_3$ | $\alpha$-Al$_2$O$_3$ | 5-Al$_2$O$_3$ | 5-Al$_2$O$_3$ | 5-Al$_2$O$_3$ | $\alpha$-Al$_2$O$_3$ | $\alpha$-Al$_2$O$_3$ | $\alpha$-Al$_2$O$_3$ |
| R (or) | / | / | 0,91 | 0,49 | 1,12 | 0,95 | / | 0,90 |
| taille or (nm) | / | / | 30 | 4 | 2,6 | 3,1 | / | 30 |
| Dispersion or (%) | / | / | 4 | 33 | 44 | 40 | / | 4 |
| Or/ Palladium | / | / | 33 | 28 | 32 | 26 | / | 40 |
| Pd croute (nm) | 80% (250$\mu$m) | 80% (250$\mu$m) | 80% (250$\mu$m) | 80% (250$\mu$m) | 80% (250$\mu$m) | 80% (250$\mu$m) | 80% (250$\mu$m) | 80% (250$\mu$m) |

On observe que seule une préparation combinant une imprégnation d'un précurseur de palladium, une imprégnation d'un précurseur d'or, suivie d'une étape de maturation, et d'une mise en contact avec de l'urée par lavage permet d'obtenir un catalyseur comprenant des particules d'or de petites tailles avec une très bonne dispersion et distribuée de manière homogène dans les grains de support poreux, tout en obtenant une répartition du palladium dans une croûte à la périphérie dudit support poreux.

Exemple 9 : Utilisation des catalyseurs C1, C2, C3, C4, C5, C6 et C8 pour l'hydrogénation sélective de la coupe C2 en configuration « Front end ».

Une charge comprenant 0,31% d'acétylène, 40% d'éthylène, 6% poids d'éthane, 30% de méthane, 16% d'hydrogène et une teneur en CO de 250 ppm mol et le complément en azote est traitée avec les catalyseurs C1, C2, C3, C4, C5, C6 et C8.

Avant la réaction, les catalyseurs C1, C2, C3, C4, C5, C6 et C8 sont activés sous un flux d'hydrogène pur à 160°C pendant 2 heures. 5 mL de catalyseurs sont placés dans un réacteur tubulaire en mode down flow. La pression du réacteur est maintenue à 3 MPa. Une vitesse horaire volumique (GHSV) de 3 000 h$^{-1}$ est appliquée. La composition de la charge et de l'effluent sont mesurées en continu en sortie du réacteur par chromatographie en phase gaz.

Les performances sont exprimées en température T1, définie comme la température nécessaire pour atteindre une conversion d'acétylène de 98%. Les températures T1 des catalyseurs C1, C2, C3, C4, C5, C6 et C8 sont reportées dans le Tableau 2 (ci-après).

La température du réacteur est maintenue constante à la température T1. La teneur en CO dans la charge est brusquement modifiée. La concentration en CO diminue de 250 ppm molaire à 80 ppm molaire. La teneur en CO dans les effluents est mesurée en sortie de réacteur. On note « t » le temps nécessaire pour que la teneur en CO mesurée en sortie de réacteur soit inférieure à 150 ppm molaire. Les valeurs de t des différents catalyseurs sont résumées dans le Tableau 2 (ci-après).

Tableau 2

| Catalyseur | Température (°C) | temps « t » (en min) pour obtenir une teneur en CO inférieure à 150 ppm molaire |
|---|---|---|
| C1 (non conforme) | 43 | < 1 |
| C2 (non conforme) | 51 | < 1 |
| C3 (non conforme) | 42 | 2 |
| C4 (non conforme) | 47 | 4 |
| C5 (conforme) | 50 | 9 |
| C6 (conforme) | 55 | 8 |
| C8 (non-conforme) | 51 | 2 |

Pour les tests menés avec les catalyseurs monométalliques Pd/Al$_2$O$_3$ C1 et C2 (non conforme), le temps nécessaire pour observer une teneur en CO dans les effluents de 150 ppm mol est très faible, inférieure à une minute.

Lorsque de l'or est présent dans le catalyseur, la durée est augmentée.

Pour les catalyseurs non-conformes à l'invention, C3, C4 et C8, la durée est comprise entre 2 et 4 minutes respectivement.

Les catalyseurs C5 et C6, conformes à l'invention, et contenant de l'or dispersé à l'échelle nanométrique et reparti de manière homogène dans le support de catalyseur, et contenant du palladium réparti dans une croûte à la périphérie du support poreux sont ceux qui permettent d'observer une teneur en CO inférieure à 150 ppm mol avec la durée la plus longue. Les catalyseurs selon l'invention jouent donc un rôle de tampon de CO et évitent des variations brusques de CO au contact desdits catalyseurs. Ils permettent ainsi d'avoir des performances catalytiques améliorées pour l'hydrogénation sélective de la coupe C2 en configuration « Front end ».

**Revendications**

1. Catalyseur comprenant de l'or, du palladium, un support poreux, se présentant sous la forme d'au moins un grain, se présentant sous forme de billes ou d'extrudés, dans lequel :

   - la teneur en or dans le catalyseur est comprise entre 0,5 et 3% poids par rapport au poids total du catalyseur ;
   - la taille moyenne des particules d'or estimée par microscopie électronique à transmission (MET) est comprise entre 0,5 nm et 5 nm ;
   - l'or est réparti de manière homogène dans ledit support poreux ; la répartition de l'or étant homogène lorsque le coefficient de répartition R est compris entre 0,8 et 1,2 ; telle que mesurée pas microsonde de Castaing, R répondant à la formule mathématique suivante :

$$R = \int_{-r}^{r} c(x)x^2\,dx \bigg/ \frac{r^2}{3}\int_{-r}^{r} c(x)\,dx$$

où

c(x) pour x∈[-r;+r] corresponds à la concentration locale en élément,
r le rayon de la bille ou de l'extrudé et
x la position du point d'analyse le long du diamètre du grain par rapport au centre de ce grain ;- au moins 80% poids du palladium est réparti dans une croûte à la périphérie dudit support poreux, l'épaisseur de ladite croûte étant inférieure à 300 μm ;

- le ratio molaire or/palladium est supérieur à 2.

2. Catalyseur selon la revendication 1, **caractérisé en ce que** la taille moyenne des particules d'or estimée par microscopie électronique à transmission est comprise entre 0,5 nm et à 3 nm.

3. Catalyseur selon les revendications 1 ou 2, **caractérisé en ce que** la dispersion métallique D de l'or est comprise entre 30 et 100 %.

4. Catalyseur selon l'une quelconque des revendications 1 à 3, **caractérisé en ce que** la teneur en palladium est comprise entre 0,01 et 0,6% poids par rapport au poids total du catalyseur.

5. Procédé de préparation d'un catalyseur selon l'une quelconque des revendications 1 à 4 lequel procédé comprenant les étapes suivantes :

- une étape dans laquelle on introduit le palladium sur le support, dite étape 1, comprenant les étapes suivantes :

1a) on prépare une solution aqueuse d'oxyde de palladium ou d'hydroxyde de palladium ;
1b) on imprègne ladite solution sur au moins un grain de support poreux ;
1c) on soumet le support poreux imprégné obtenu à l'étape 1b) à une maturation afin d'obtenir un précurseur de catalyseur, pendant une durée comprise entre 0,5 et 40 heures ;
1d) on sèche le précurseur de catalyseur obtenu à l'étape 1c) à une température comprise entre 50°C et 250°C ;
1e) on calcine le précurseur de catalyseur obtenu à l'étape 1d) à une température comprise entre 250°C et 900°C ;

- une étape dans laquelle on introduit l'or sur le support, dite étape 2, comprenant les étapes suivantes :

2a) on prépare une solution aqueuse contenant un précurseur d'or ;
2b) on imprègne ledit support poreux avec la ladite solution obtenue à l'étape 2a);
2c) on soumet le support poreux imprégné obtenu à l'étape 2b) à une maturation afin d'obtenir un précurseur de catalyseur, pendant une durée comprise entre 0,5 et 40 heures ;
2d) on met en contact le précurseur de catalyseur obtenu à l'étape 2c) avec une solution aqueuse contenant de l'urée, le volume de la solution aqueuse contenant l'urée étant compris entre 0,9 et 20 fois le volume poreux du catalyseur mis en forme, et le rapport molaire urée/or étant compris entre 1 et 1000 ;
2e) on sèche le précurseur de catalyseur obtenu à l'étape 2d) à une température comprise entre 50°C et 300°C pendant une durée comprise entre 0,5 et 20 heures ;

les étapes 1 et 2 étant effectuées dans un ordre quelconque, étant entendu que lorsque l'étape 2 est effectuée avant l'étape 1, ledit procédé comprend en outre une étape 2f) dans lequel on soumet le catalyseur séché obtenu à l'issue de l'étape 2e) à un traitement réducteur par mise en contact avec un gaz réducteur.

6. Procédé selon la revendication 5, dans laquelle on prépare à l'étape 1a) une suspension colloïdale d'oxyde de palladium ou d'hydroxyde de palladium en phase aqueuse.

7. Procédé selon les revendications 5 ou 6, dans laquelle on réalise à l'étape 1b) et/ou 2b) une imprégnation à sec.

8. Procédé selon l'une quelconque des revendications 5 à 7, **caractérisé en ce que** l'étape 1c) et/ou 2c) de maturation est réalisée pendant une durée comprise entre 1 et 30 heures

9. Procédé selon les revendications 5 à 8, **caractérisé en ce qu'**on réalise entre l'étape 2c) et 2d) une étape de séchage dudit précurseur de catalyseur obtenu à l'étape 2c) à une température comprise entre 50°C et 300°C.

10. Procédé selon l'une quelconque des revendications 5 à 9, **caractérisé en ce que** le rapport molaire urée/or est compris entre 2 et 700, de manière très préférée entre 3 et 300.

11. Procédé selon la revendication 5, **caractérisé en ce que** l'étape 2f) de réduction est réalisée à une température comprise entre 40°C et 500°C.

12. Procédé d'hydrogénation sélective d'une coupe C2-C4 avec un catalyseur selon l'une quelconque des revendications 1 à 4 ou préparé selon l'une quelconque des revendications 5 à 11 dans lequel la température est comprise entre 0 et 500°C, la pression est comprise entre 0,1 et 20 MPa, la vitesse volumique horaire est comprise entre 0,1 et 50 $h^{-1}$ pour une charge liquide, entre 500 et 30 000 $h^{-1}$ pour une charge gazeuse.

**Patentansprüche**

1. Katalysator, umfassend Gold, Palladium, einen porösen Träger in Form mindestens eines Korns, in Form von Kugeln oder Extrudaten, wobei:

   - der Goldgehalt im Katalysator im Bereich von 0,5 bis 3 Gew.-%, bezogen auf das Gesamtgewicht des Katalysators, liegt;
   - die mittels Transmissionselektronenmikroskopie (TEM) bestimmte mittlere Größe der Goldpartikel im Bereich von 0,5 nm bis 5 nm liegt;
   - das Gold in dem porösen Träger homogen verteilt ist, wobei die Verteilung des Goldes homogen ist, wenn der Verteilungskoeffizient R im Bereich von 0,8 bis 1,2 liegt;
   wobei R, wie mittels Castaing-Mikrosonde gemessen, der folgenden mathematischen Formel entspricht:

   $$R = \int_{-r}^{r} c(x)x^2\,dx \Big/ \frac{r^2}{3}\int_{-r}^{r} c(x)dx \quad,$$

   worin:

   $c(x)$ für $x \in [-r;+r]$ der lokalen Elementkonzentration entspricht,
   r der Radius der Kugel oder des Extrudats ist und
   x die Position des Analysepunkts entlang des Durchmessers des Korns, bezogen auf die Mitte des Korns, ist;

   - mindestens 80 Gew.-% Palladium in einer Rinde an der Peripherie des porösen Trägers verteilt sind, wobei die Dicke der Rinde kleiner als 300 $\mu$m ist;
   - das Molverhältnis Gold/Palladium höher als 2 ist.

2. Katalysator nach Anspruch 1, **dadurch gekennzeichnet, dass** die mittels Transmissionselektronenmikroskopie bestimmte mittlere Größe der Goldpartikel im Bereich von 0,5 nm bis 3 nm liegt.

3. Katalysator nach den Ansprüchen 1 oder 2, **dadurch gekennzeichnet, dass** die metallische Dispersion D des Goldes im Bereich von 30% bis 100% liegt.

4. Katalysator nach einem der Ansprüche 1 bis 3, **dadurch gekennzeichnet, dass** der Palladiumgehalt im Bereich von 0,01 bis 0,6 Gew.-%, bezogen auf das Gesamtgewicht des Katalysators, liegt.

5. Verfahren zur Herstellung eines Katalysators nach einem der Ansprüche 1 bis 4, wobei das Verfahren die folgenden Schritte umfasst:

   - einen Schritt, in dem man das Palladium auf den Träger überführt, der als Schritt 1 bezeichnet wird und die folgenden Schritte umfasst:

      1a) man stellt eine wässrige Lösung von Palladiumoxid oder Palladiumhydroxid her;

1b) man imprägniert mindestens ein Korn des porösen Trägers mit dieser Lösung;

1c) man unterzieht den in Schritt 1b) erhaltenen imprägnierten porösen Träger einer Reifung für eine Dauer im Bereich von 0,5 bis 40 Stunden, um einen Katalysatorvorläufer zu erhalten;

1d) man trocknet den in Schritt 1c) erhaltenen Katalysatorvorläufer bei einer Temperatur im Bereich von 50°C bis 250°C;

1e) man kalziniert den in Schritt 1d) erhaltenen Katalysatorvorläufer bei einer Temperatur im Bereich von 250°C bis 900°C;

- einen Schritt, in dem man das Gold auf den Träger überführt, der als Schritt 2 bezeichnet wird und die folgenden Schritte umfasst:

2a) man stellt eine wässrige Lösung her, die einen Goldvorläufer enthält;

2b) man imprägniert den porösen Träger mit der in Schritt 2a) erhaltenen Lösung;

2c) man unterzieht den in Schritt 2b) erhaltenen imprägnierten porösen Träger einer Reifung für eine Dauer im Bereich von 0,5 bis 40 Stunden, um einen Katalysatorvorläufer zu erhalten;

2d) man bringt den in Schritt 2c) erhaltenen Katalysatorvorläufer mit einer wässrigen Lösung in Kontakt, die Harnstoff enthält, wobei das Volumen der wässrigen, Harnstoff-haltigen Lösung im Bereich des 0,9- bis 20-Fachen des Porenvolumens des geformten Katalysators liegt und das Molverhältnis Harnstoff/Gold im Bereich von 1 bis 1000 liegt;

2e) man trocknet den in Schritt 2d) erhaltenen Katalysatorvorläufer bei einer Temperatur im Bereich von 50°C bis 300°C für eine Dauer im Bereich von 0,5 bis 20 Stunden;

wobei die Schritte 1 und 2 in beliebiger Reihenfolge durchgeführt werden, wobei selbstverständlich ist, dass, wenn Schritt 2 vor Schritt 1 durchgeführt wird, das Verfahren ferner einen Schritt 2f) umfasst, in dem man den nach Abschluss von Schritt 2e) erhaltenen getrockneten Katalysator einer Reduktionsbehandlung durch Inkontaktbringen mit einem reduzierenden Gas unterzieht.

6. Verfahren nach Anspruch 5, wobei man in Schritt 1a) eine kolloidale Suspension von Palladiumoxid oder Palladiumhydroxid in einer wässrigen Phase herstellt.

7. Verfahren nach den Ansprüchen 5 oder 6, wobei man in Schritt 1b) und/oder 2b) eine Trockenimprägnierung durchführt.

8. Verfahren nach einem der Ansprüche 5 bis 7, **dadurch gekennzeichnet, dass** der Reifungsschritt 1c) und/oder 2c) für eine Dauer im Bereich von 1 bis 30 Stunden durchgeführt wird.

9. Verfahren nach den Ansprüchen 5 bis 8, **dadurch gekennzeichnet, dass** man zwischen Schritt 2c) und 2d) einen Schritt des Trocknens des in Schritt 2c) erhaltenen Katalysatorvorläufers bei einer Temperatur im Bereich von 50°C bis 300°C durchführt.

10. Verfahren nach einem der Ansprüche 5 bis 9, **dadurch gekennzeichnet, dass** das Molverhältnis Harnstoff/Gold im Bereich von 2 bis 700, sehr stark bevorzugt von 3 bis 300, liegt.

11. Verfahren nach Anspruch 5, **dadurch gekennzeichnet, dass** der Reduktionsschritt 2f) bei einer Temperatur im Bereich von 40°C bis 500°C durchgeführt wird.

12. Verfahren zur selektiven Hydrierung eines C2-C4-Schnitts mit einem Katalysator nach einem der Ansprüche 1 bis 4 oder hergestellt nach einem der Ansprüche 5 bis 11, wobei die Temperatur im Bereich von 0 bis 500°C liegt, der Druck im Bereich von 0,1 bis 20 MPa liegt, die Raumstundengeschwindigkeit im Bereich von 0,1 bis 50 $h^{-1}$ für eine flüssige Beschickung und im Bereich von 500 bis 30000 $h^{-1}$ für eine gasförmige Beschickung liegt.

## Claims

1. Catalyst comprising gold, palladium and a porous support, which is in the form of at least one grain, which is in the form of beads or extrudates, in which:

- the gold content in the catalyst is between 0.5 and 3% by weight relative to the total weight of the catalyst;
- the mean size of the gold particles estimated by transmission electron microscopy (TEM) is between 0.5 nm and 5 nm;
- the gold is homogeneously distributed in said porous support; the gold distribution being homogeneous when the partition coefficient R is between 0.8 and 1.2; as measured by a castaing microprobe R corresponding to the following mathematical formula:

$$R = \int_{-r}^{r} c(x)x^2 dx \bigg/ \frac{r^2}{3} \int_{-r}^{r} c(x)dx$$

wherein
change pour to for

c(x) for $x \in [-r,+r]$ corresponds to the local concentration of element,
r the radius of the bead or of the extrudant and
x the position of the point of analysis along the diameter of the grain relative to the centre of this grain;
at least 80% by weight of the palladium is distributed in a crust at the periphery of said porous support, the thickness of said crust being less than 300 $\mu$m;

- the gold/palladium molar ratio is greater than 2.

2. Catalyst according to Claim 1, **characterized in that** the mean size of the gold particles estimated by transmission electron microscopy is between 0.5 and 3 nm.

3. Catalyst according to Claims 1 or 2, **characterized in that** the metal dispersion D of the gold is between 30 and 100%.

4. Catalyst according to any one of Claims 1 to 3, **characterized in that** the palladium content is between 0.01 and 0.6% by weight relative to the total weight of the catalyst.

5. Method for preparing a catalyst according to any one of Claims 1 to 4, which method comprises the following steps:

- a step in which the palladium is introduced onto the support, termed step 1, comprising the following steps:

1a) an aqueous solution of palladium oxide or of palladium hydroxide is prepared;
1b) said solution is impregnated onto at least one grain of porous support;
1c) the impregnated porous support obtained in step 1b) is subjected to maturation in order to obtain a catalyst precursor, for a period of between 0.5 and 40 hours;
1d) the catalyst precursor obtained in step 1c) is dried at a temperature of between 50°C and 250°C;
1e) the catalyst precursor obtained in step 1d) is calcined at a temperature of between 250°C and 900°C;

- a step in which the gold is introduced onto the support, termed step 2), comprising the following steps:

2a) an aqueous solution containing a gold precursor is prepared;
2b) said porous support is impregnated with said solution obtained in step 2a);
2c) the impregnated porous support obtained in step 2b) is subjected to maturation in order to obtain a catalyst precursor, for a period of between 0.5 and 40 hours;
2d) the catalyst precursor obtained in step 2c) is brought into contact with an aqueous solution containing urea, the volume of the aqueous solution containing urea being between 0.9 and 20 times the pore volume of the catalyst formed, and the urea/gold molar ratio being between 1 and 1000;
2e) the catalyst precursor obtained in step 2d) is dried at a temperature of between 50°C and 300°C for a period of between 0.5 and 20 hours;

steps 1 and 2 being carried out in any order, it being understood that, when step 2 is carried out before step 1, said method also comprises a step 2f) in which the dried catalyst obtained at the end of step 2e) is subjected to a reducing treatment by bringing into contact with a reducing gas.

6. Method according to Claim 5, in which a colloidal suspension of palladium oxide or of palladium hydroxide in the aqueous phase is prepared in step 1a).

7. Method according to Claim 5 or 6, in which a dry impregnation is carried out in step 1b) and/or 2b) .

8. Method according to any one of Claims 5 to 7, **characterized in that** the maturation step 1c) and/or 2c) is carried out for a period of between 1 and 30 hours.

9. Method according to Claims 5 to 8, **characterized in that** a step of drying said catalyst precursor obtained in step 2c) at a temperature of between 50°C and 300°C is carried out between step 2c) and 2d) .

10. Method according to any one of Claims 5 to 9, **characterized in that** the urea/gold molar ratio is between 2 and 700, very preferably between 3 and 300.

11. Method according to Claim 5, **characterized in that** the reduction step 2f) is carried out at a temperature of between 40°C and 500°C.

12. Method for selective hydrogenation of a C2-C4 fraction with a catalyst according to any one of Claims 1 to 4 or prepared according to any one of Claims 5 to 11,
in which the temperature is between 0 and 500°C, the pressure is between 0.1 and 20 MPa, and the hourly space velocity is between 0.1 and 50 h$^{-1}$ for a liquid feedstock, between 500 and 300 000 h$^{-1}$ for a gaseous feedstock.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2006025302 A
- US 6509292 B
- US 6350717 B
- FR 2347095
- FR 2932177

**Littérature non-brevet citée dans la description**

- **PAVANI MALLA.** Designing supported palladium-on-gold bimetallic nano-catalysts for controlled hydrogenation of acetylene in large excess of ethylene. *Texas A&M University,* Août 2014
- **D.R. LIDE.** CRC Handbook of Chemistry and Physics. 2000
- **ROUQUEROL F. ; ROUQUEROL J. ; SINGH K.** Adsorption by Powders & Porous Solids: Principle, methodology and applications. Academic Press, 1999
- **R. VAN HARDEVELD ; F. HARTOG.** The statistics of surface atoms and surface sites on metal crystals». *Surface Science,* 1969, vol. 15, 189-230